# EUROPEAN PATENT APPLICATION

(11) **EP 4 834 255 A1**
(43) Date of publication of application: **04.11.2026**
(21) Application number: 24911902.5
(22) Date of filing: 02.10.2024
(51) Int. Cl.: A61B 17/3213, B26B 1/08

(54) **MEDICAL EDGED TOOL**

(30) Priority: 27.12.2023 JP 2023221197
(71) Applicant: KAI R&D CENTER CO., LTD., Seki-shi, Gifu 501-3992 (JP)
(72) Inventor: SATO, Yuki, Seki-shi, Gifu 501-3992 (JP)
(74) Representative: Plasseraud IP
(86) International application number: PCT/JP2024/035220
(87) International publication number: WO 2025/142008

(57) **Abstract**

A medical knife according to the present invention sandwiches a blade body with a holder and a cover, in which the blade body has, in plan view, a blade edge extending from a tip, and having a cutting edge at least partially, a spine extending from the tip, and a first edge portion extending in a direction having a first angle with respect to the spine, the holder has a blade-body holding recessed portion having a recessed shape and formed along a shape of the blade body, and the blade-body holding recessed portion has a first slanted portion slanted in a direction perpendicular to the first edge portion in plan view at a position where the first edge portion abuts, and a second slanted portion slanted in a direction perpendicular to the spine in plan view at a position where the spine abuts.

## Description

### Technical Field

One aspect of the present invention relates to a medical knife.

### Background Art

Conventionally, there has been a scalpel (medical knife) that includes a handle, a blade body, and a slider (movable body), and enables retraction of the blade body by holding the handle by hand and moving the slider to which the blade body is attached. Such a scalpel has a configuration in which the handle formed of plastic and the slider are integrated, but because the slider moves inside the handle, play may develop between the slider and the handle. At this time, because the blade body is attached to the slider, the blade body wobbles against the handle.

On the other hand, a medical knife described in Patent Literature 1 includes a movable body including a handle, a blade body, and a cover. In this configuration, because the blade body is attached to the handle, the blade body itself does not wobble even if play develops between the handle and the movable body.

In addition, there is another medical knife described in Patent Literature 2.

### Citation List

### Patent Literature

Patent Literature 1: JP 2017-86590 A
Patent Literature 2: JP 2018-143322 A

### Summary of Invention

### Technical Problem

However, in the medical knife described in Patent Literature 1, the blade body is sandwiched by blade-body overlapping portions of the handle formed of plastic, and play may develop between a resin member and the blade body. More specifically, each of the two blade-body overlapping portions has a protruding portion and a recessed portion that are slightly slanted, by which the blade-body overlapping portions sandwich the blade body such that a rear end portion of the blade body is slightly warped, but the play may develop in a width direction of the blade body. In particular, a medical knife for performing precise work is required to be capable of preventing such slight play.

In addition, in Goal 3 among the 17 Sustainable Development Goals (SDGs) adopted at the United Nations Summit, "Ensure healthy lives and promote well-being for all at all ages." is mentioned as the goal of "Good Health and Well-Being". Under such circumstances, it is also required to supply an instrument that is safer and easier to use for instruments used in the medical field.

### Solution to Problem

In order to solve the above problem, the present invention provides the following means, for example.

A medical knife according to one aspect of the present invention sandwiches a blade body with a holder and a cover, in which
the blade body has, in plan view,
a blade edge extending from a tip, and having a cutting edge at least partially,
the spine extending from the tip, and
a first edge portion extending in a direction having a first angle with respect to the spine,
the holder has a blade-body holding recessed portion having a recessed shape and formed along a shape of the blade body, and
the blade-body holding recessed portion has
a first slanted portion slanted in a direction perpendicular to the first edge portion in plan view at a position where the first edge portion abuts, and
a second slanted portion slanted in a direction perpendicular to the spine in plan view at a position where the spine abuts.

According to the medical knife described above, the blade body is sandwiched between the holder and the cover while being curved in a thickness direction by the first slanted portion and second slanted portion of the blade-body holding recessed portion of the holder, and thus it is possible to prevent wobble in a longitudinal direction, width direction, and rotation direction of the blade body with a relatively simple configuration. Accordingly, it is possible to provide the medical knife capable of accurately incising a target site or the like. By using such a medical knife, it is possible to provide an instrument that is safe and easy to use, and it is possible to contribute to Goal 3 of SDGs "Good Health and Well-Being".

In the medical knife described above, the blade body preferably further has, in plan view, a second edge portion extending in a direction having a second angle with respect to the spine, from an end portion of the spine, the end portion being on a side opposite to the tip, and
the first edge portion preferably extends, in plan view, from the second edge portion toward the blade edge, in a direction having a third angle with respect to the second edge portion.

According to the medical knife described above, the second slanted portion and the first slanted portion are provided on the spine and the first edge portion connected to the second edge portion continuing from the spine, respectively, and thus the blade body can be sandwiched more stably by the holder and the cover.

In the medical knife described above, the third angle is preferably 90°.

In the medical knife described above, the blade body preferably has a through hole having an elongated hole shape extending parallel with a direction in which the spine extends, and formed in a thickness direction,
the holder preferably has a blade-body insertion portion having a protruding shape and being inserted into the through hole, and
in plan view, the through hole is preferably formed so as to intersect a straight line connecting a central portion in a direction in which the first slanted portion extends and a central portion in a direction in which the second slanted portion extends.

According to the medical knife described above, the straight line connecting the central portion in the direction in which the first slanted portion extends and the central portion in the direction in which the second slanted portion extends intersects the through hole through which the blade-body insertion portion is inserted. Accordingly, the blade body can be more stably held with respect to the holder, and thus wobble of the blade body can be more effectively prevented.

### Brief Description of Drawings

[Figure 1]Fig. 1 is a perspective view of a medical knife as viewed from a first direction.
[Figure 2]Fig. 2 is a perspective view of the medical knife in which a blade body is exposed by moving a slider, as viewed from the first direction.
[Figure 3]Fig. 3 is a perspective view of the medical knife from which the slider is removed, as viewed from the first direction.
[Figure 4]Fig. 4 is a perspective view of the medical knife from which the slider and a cover are removed, as viewed from the first direction.
[Figure 5]Fig. 5 is an enlarged perspective view of the medical knife from which the slider, the cover, and the blade body are removed and only a holder is left, as viewed from the first direction.
[Figure 6]Fig. 6 is an enlarged perspective view of the holder of the medical knife as viewed from a second direction.
[Figure 7]Fig. 7 is a perspective view of the medical knife as viewed from the second direction.
[Figure 8]Fig. 8 is a perspective view of the medical knife as viewed from a third direction.
[Figure 9]Fig. 9 is a perspective view of the medical knife from which the slider is removed, as viewed from the third direction.
[Figure 10]Fig. 10 is a perspective view of the cover and blade body of the medical knife, as viewed from the third direction.
[Figure 11]Fig. 11 is a perspective view of the cover of the medical knife, as viewed from the third direction.
[Figure 12]Fig. 12 is a side view of the medical knife.
[Figure 13]Fig. 13 is a side view of the medical knife in which the blade body is exposed by moving the slider.
[Figure 14]Fig. 14 is a side view of the medical knife from which the slider is removed.
[Figure 15]Fig. 15 is an enlarged plan view of the blade body placed on the holder.
[Figure 16]Fig. 16 is a plan view of the medical knife from which the slider is removed.
[Figure 17]Fig. 17 is a cross-sectional view taken along line A-A in Fig. 16.

### Description of Embodiments

A medical knife according to an embodiment of the present invention will be specifically described with reference to the drawings. The medical knife of the present invention is a so-called medical scalpel. The embodiment described below is merely an example of the present invention, and does not limit the technical scope of the present invention. Note that in the drawings, the same components are denoted by the same reference signs, and redundant description is omitted.

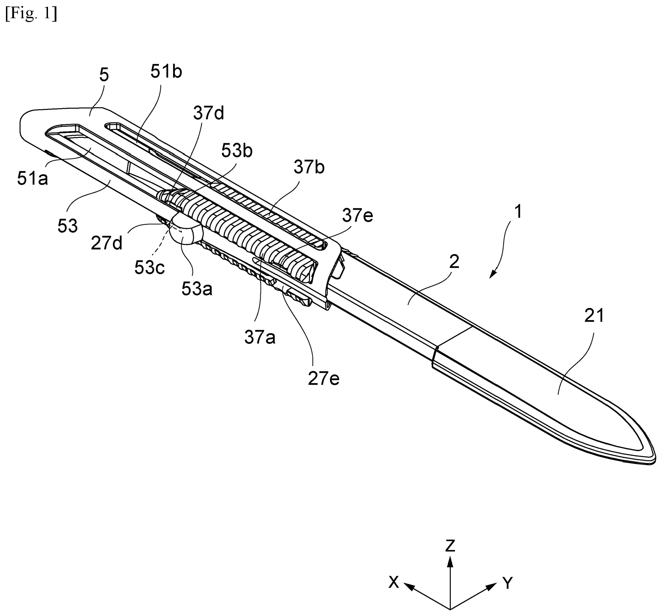

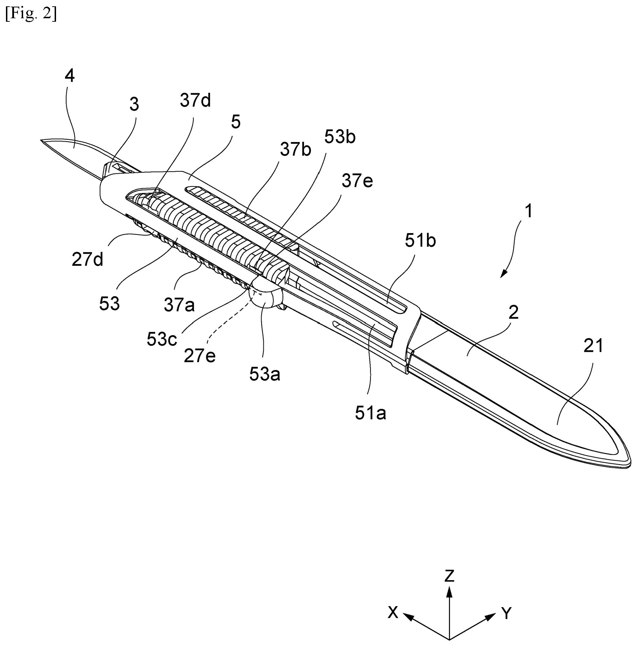

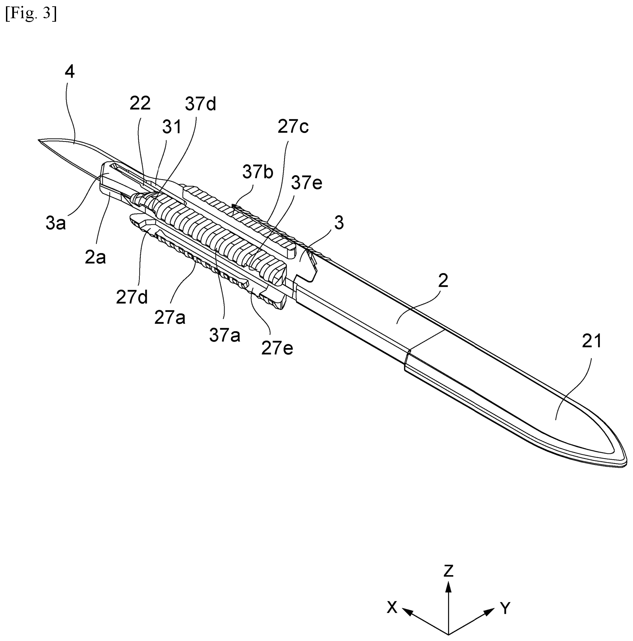

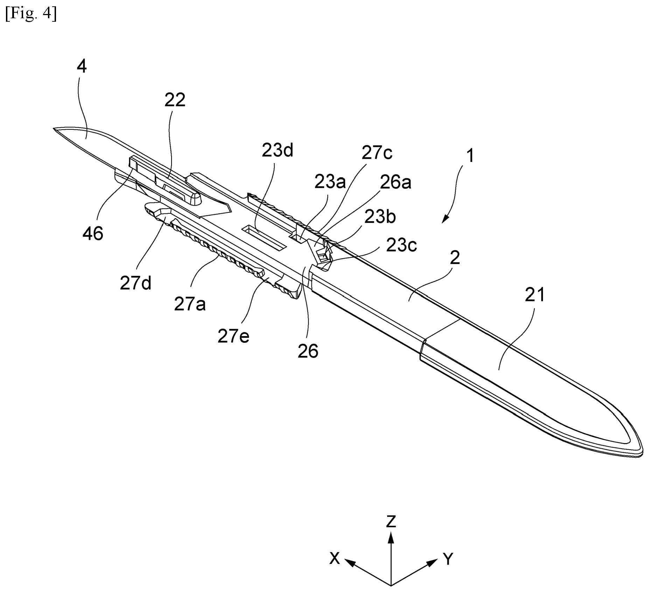

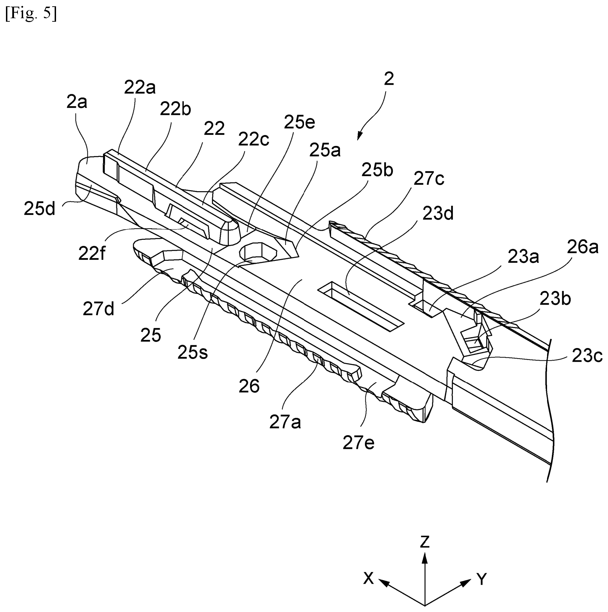

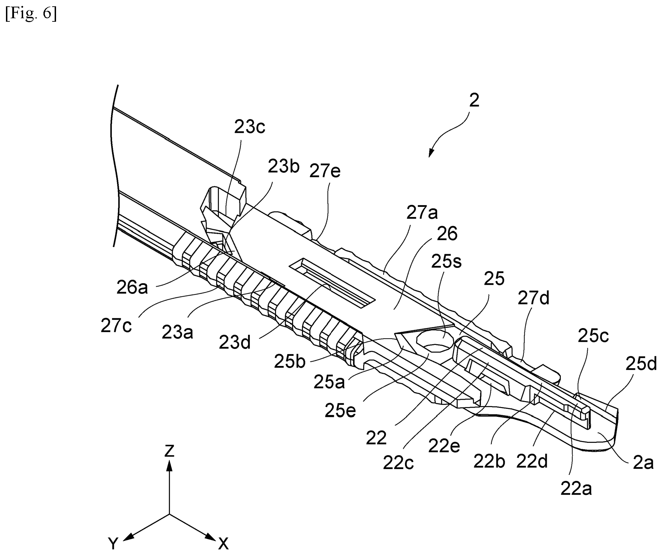

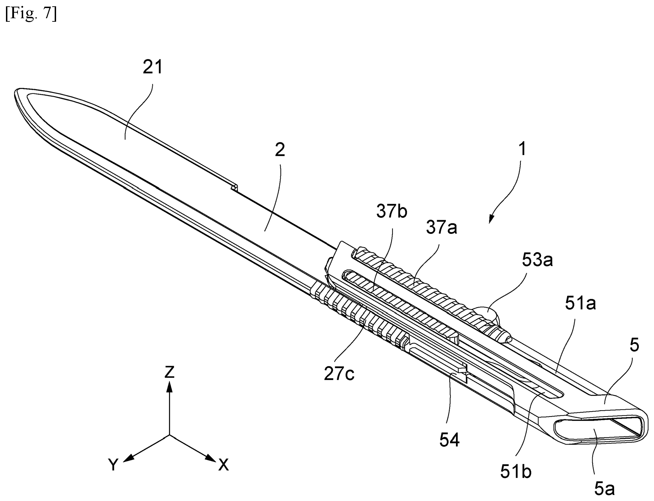

Figs. 1 to 5 are perspective views of a medical knife 1 as viewed from a first direction. Fig. 1 shows a state in which a blade body 4 is covered with a slider 5, Fig. 2 shows a state in which the blade body is exposed by moving the slider 5, Fig. 3 shows a state in which the slider 5 is removed, and Fig. 4 shows a state in which a cover 3 is further removed. That is, Fig. 4 is a perspective view of a holder 2 and the blade body 4 of the medical knife 1. Fig. 5 is an enlarged view of the medical knife 1 from which the slider 5, the cover 3, and the blade body 4 are removed and only the holder 2 is left. Fig. 6 is an enlarged perspective view of the holder 2 shown in Fig. 5, as viewed from a second direction different from the first direction. Fig. 7 is perspective view of the medical knife 1 as viewed from the second direction.

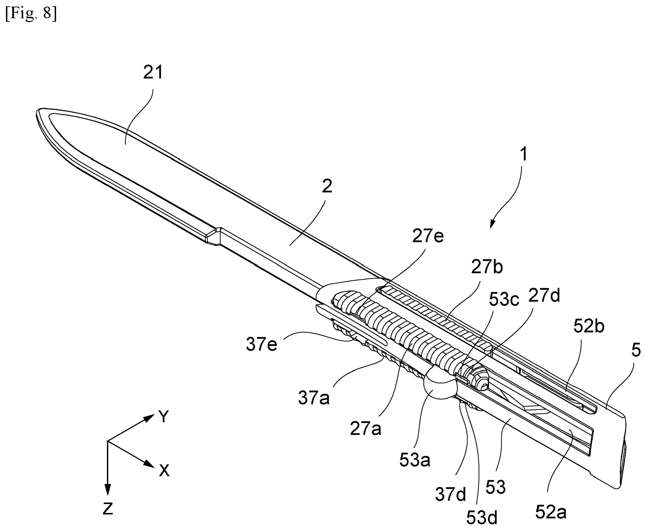

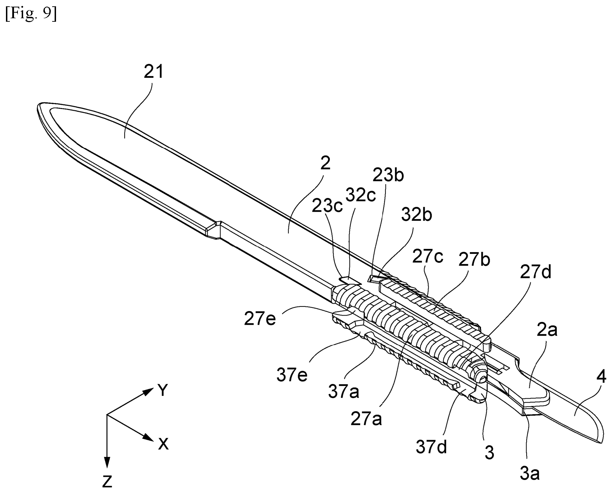

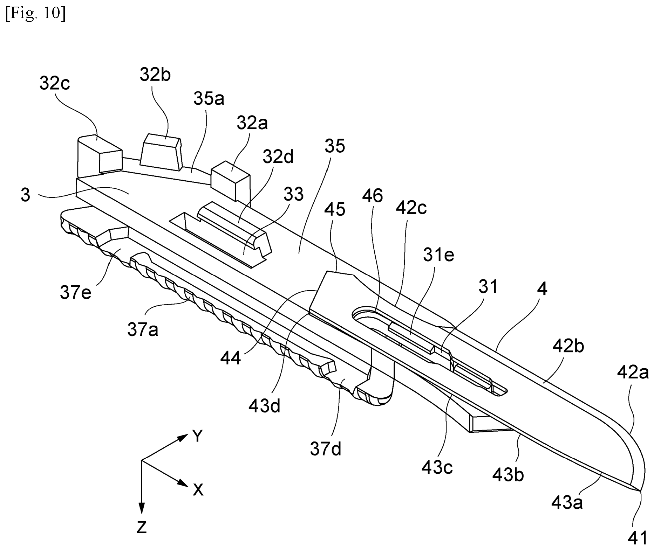

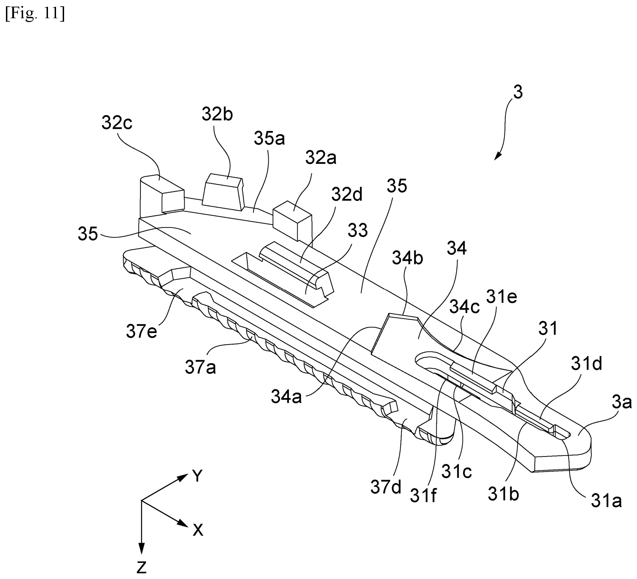

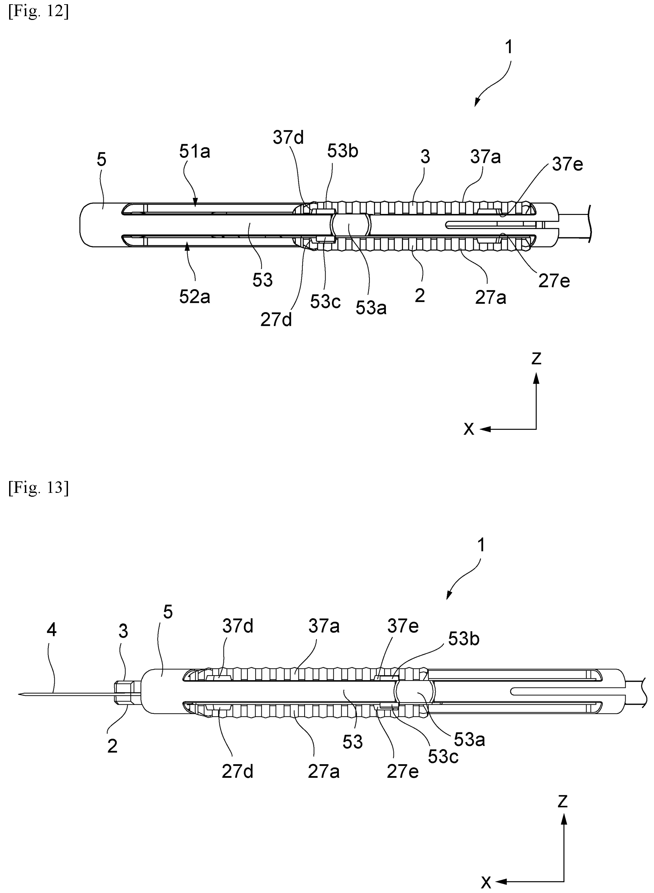

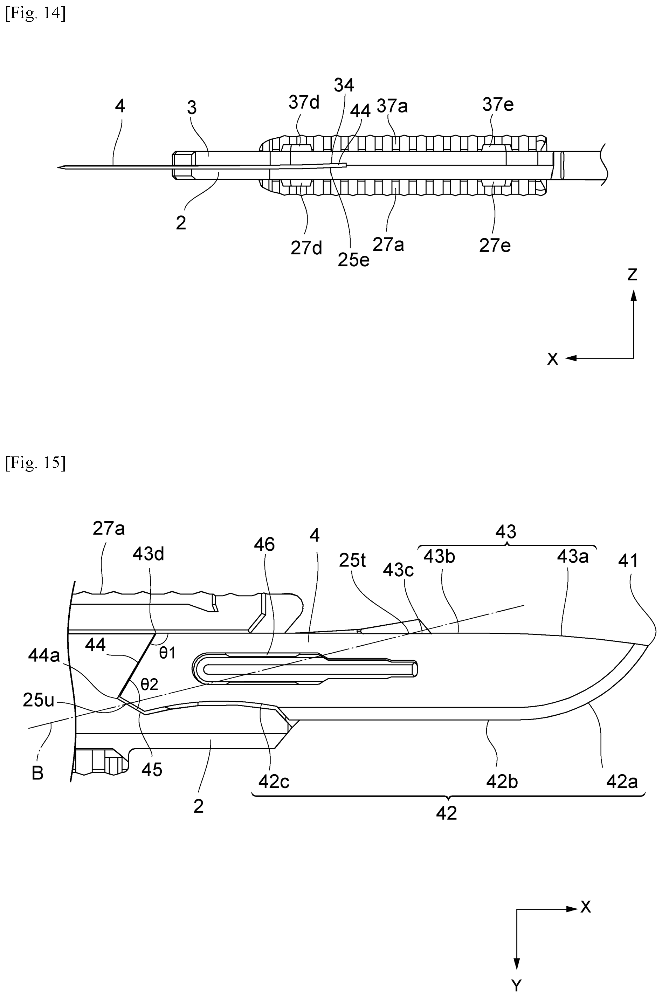

Figs. 8 to 11 are perspective views of the medical knife 1 as viewed from a third direction. Fig. 8 shows a state in which the blade body 4 is covered with the slider 5, Fig. 9 shows a state in which the slider 5 is removed, Fig. 10 shows a state in which the holder 2 is further removed, and Fig. 11 shows a state in which the blade body 4 is further removed. That is, Fig. 11 is a view showing the cover 3 of the medical knife 1. Figs. 12 to 14 are side views of the medical knife 1. Fig. 12 shows a state in which the blade body 4 is covered with the slider 5, Fig. 13 shows a state in which the blade body 4 is exposed by moving the slider 5, and Fig. 14 shows a state in which the slider 5 is removed.

In the present specification, a state in which the holder 2, the cover 3, the blade body 4, and the slider 5 are connected may be referred to as a connected state or an assembled state.

In the drawings, an X axis, a Y axis, and a Z axis are shown. A direction in which the blade body 4 extends is an X-axis direction, a thickness direction of the medical knife 1 is a Z-axis direction, and the X axis, the Y axis, and the Z axis are directions perpendicular to one another.

As shown in each drawing, the medical knife 1 of the present embodiment includes the holder 2, the cover 3, the blade body 4, and the slider 5. The holder 2, the cover 3, and the slider 5 are formed of resin, and the blade body 4 is formed of metal. The medical knife 1 sandwiches the blade body 4 with the holder 2 and the cover 3 (see Fig. 3, Fig. 9, Fig. 14, and the like). The slider 5 is slidably attached to the holder 2 and the cover 3 in the connected state. As the slider 5 slides, a state of the medical knife 1 changes between a housed state (see Fig. 1) in which the blade body 4 is covered by the slider 5 so as not to be exposed, and a use state (see Fig. 2) in which the blade body 4 is exposed.

### [Blade body 4]

Fig. 15 is an enlarged plan view of the blade body 4 placed on the holder 2. The blade body 4 is formed of a plate-like metal, and has a cutting edge polished as described in detail below. The blade body 4 has a blade edge 42 extending from a tip 41, a spine 43 extending from the tip 41, a second edge portion 44 extending from the spine 43, and a first edge portion 45 extending from the second edge portion 44 toward the blade edge 42. The blade body 4 is formed with the blade edge 42, the spine 43, the second edge portion 44, and the first edge portion 45 as outer edges thereof in plan view.

The blade edge 42 extends from the tip 41 and has a polished cutting edge at least partially. The blade edge 42 has a curved blade edge 42a, a straight blade edge 42b, and a cutout portion 42c. The cutting edge is formed on the blade edge 42 over the entire curved blade edge 42a and to a position halfway through the straight blade edge 42b. The curved blade edge 42a curves outward in a protruding shape. The straight blade edge 42b is linear, and the cutout portion 42c has a shape cut out so as to curve inward in a substantially arc shape from a straight line that is an extended line of the straight blade edge 42b. In the present embodiment, the entire curved blade edge 42a and a portion of the straight blade edge 42b form the cutting edge as described above, but a portion where the cutting edge is formed may be changed.

The spine 43 extends from the tip 41 and has a curved spine 43a and a straight spine 43b. It can be said that the curved spine 43a is gently curved, the straight spine 43b extends linearly, and the spine 43 is substantially linear. In the vicinity of the center of the spine 43, there is a slanted abutting portion 43c that abuts a second slanted portion 25c of the holder 2 in the connected state in which the blade body 4 is sandwiched by the holder 2 and the cover 3.

The second edge portion 44 extends toward a blade edge 42 side from an end portion (corner portion) 43d of the spine 43, which is on a side opposite to the tip 41. The second edge portion 44 is linear. An angle θ1 formed by the straight spine 43b which is a linear portion of the spine 43 and the second edge portion 44 is 120°.

The first edge portion 45 extends toward a tip 41 side of the blade edge 42, from an end portion (corner portion) 44a of the second edge portion 44, the end portion 44a being opposite to an end portion (corner portion) 43d which is a portion connecting the second edge portion 44 and the spine 43. The first edge portion 45 is linear. An angle θ2 formed by the second edge portion 44 and the first edge portion 45 is 90°, that is, a right angle. An angle formed by the extended line of the spine 43 and an extended line of the first edge portion 45 is 30° (= θ1 + θ2-180°).

The blade body 4 has a through hole 46 formed to penetrate the blade body 4 in the thickness direction, on an inner side in planar view. The through hole 46 extends in an elongated hole shape in parallel with an X direction, which is a direction in which the spine 43 extends. More specifically, a length of the through hole 46 in a Y-axis direction, which is a width direction, changes in an order of a first width portion 46a, a second width portion 46b, and a third width portion 46c from a tip 41 side. For lengths of the widths, the first width portion 46a < the second width portion 46b < the third width portion 46c holds.

### [Holder 2]

The holder 2 is a member that is connected to the cover 3 by fitting, and sandwiches the blade body 4 together with the cover 3 in the connected state in which the holder 2 is connected to the cover 3 and the blade body 4. The holder 2 has a grip part 21 to be gripped by a user. An end portion of the grip part 21 on a side opposite to the blade body 4 in a longitudinal direction has a tapered shape that narrows in the width direction toward a tip thereof.

As shown in Figs. 4, 5, and the like, the holder 2 has a blade-body insertion portion 22 having a protruding shape and being inserted into the through hole 46 of the blade body 4 and a through hole 31 of the cover 3 in the connected state (see Fig. 10). The blade-body insertion portion 22 has a shape along the through hole 46 and the through hole 31. That is, the blade-body insertion portion 22 has a first width portion 22a, a second width portion 22b, and a third width portion 22c corresponding to the first width portion 46a, second width portion 46b, and third width portion 46c of the through hole 46. For lengths of the widths, the first width portion 22a < the second width portion 22b < the third width portion 22c holds.

The holder 2 has recessed portions 22d, 22e, and 22f that are recessed in the width direction (Y-axis direction) at a base of the blade-body insertion portion 22. The recessed portions 22e and 22f are formed on opposite sides in the Y axis direction. These recessed portions 22d, 22e, and 22f are provided at positions corresponding to protruding portions 31d, 31e, and 31f of the cover 3, and in the connected state, the protruding portions 31d, 31e, and 31f are inserted into the recessed portions 22d, 22e, and 22f, respectively, by which the holder 2 and the cover 3 are fitted to each other.

The holder 2 has a blade-body holding recessed portion 25 having a recessed shape and formed along a shape of the blade body 4. The blade-body holding recessed portion 25 is formed at a position facing the cover 3 and is formed as a surface one step lower than a cover facing surface 26 facing the cover 3 without interposing the blade body 4 therebetween (surface positioned on a negative Z-axis side). The cover facing surface 26 has a planar shape along an X-Y plane. A holder tip-end portion 2a on a tip-end side (positive X-axis side) of the blade-body holding recessed portion 25 has the same shape in plan view as a cover tip-end portion 3a of the cover 3.

As shown in Figs. 5 and 6, the blade-body holding recessed portion 25 has a first slanted portion 25a at a position where the first edge portion 45 of the blade body 4 abuts in the connected state. The first slanted portion 25a is slanted so as to be recessed inward from an edge 25b, in other words, is slanted in a direction perpendicular to a direction in which the first edge portion 45 of the blade body 4 extends, in plan view. Accordingly, in the connected state, the first edge portion 45 of the blade body 4 abuts a slanted surface of the first slanted portion 25a.

As shown in Fig. 6, the blade-body holding recessed portion 25 has the second slanted portion 25c at a position where the straight spine 43b of the blade body 4 abuts in the connected state. The second slanted portion 25c is slanted so as to be recessed inward from an edge 25d, in other words, is slanted in a direction perpendicular to a direction in which the straight spine 43b of the blade body 4 extends, in plan view. Accordingly, in the connected state, a portion of the straight spine 43b of the blade body 4 abuts a slanted surface of the second slanted portion 25c.

As shown Figs. 5, 6, and the side view in Fig. 14, the blade-body holding recessed portion 25 has a third slanted portion 25e at a position continuous from the first slanted portion 25a. The third slanted portion 25e is slanted along the longitudinal direction (X-axis direction) of the holder 2. More specifically, the third slanted portion 25e is slanted toward the positive Z-axis side as it extends toward a negative X-axis side.

As shown in Figs. 5, 6, and the like, the holder 2 has connecting hole portions 23a, 23b, 23c, and 23d. The connecting hole portion 23d is a rectangular through hole extending in the longitudinal direction (X-axis direction). The connecting hole portions 23a, 23b, and 23c are provided at positions corresponding to connecting protruding portions 32a, 32b, and 32c of the cover 3. In the connected state, the connecting protruding portions 32a, 32b, 32c, and 32d of the cover 3 are inserted into the connecting hole portions 23a, 23b, 23c, and 23d, by which the holder 2 and the cover 3 are fitted to each other.

A periphery of a position where the connecting hole portion 23b is formed is a cover-facing slanted surface 26a facing a slanted edge portion 35a of the cover 3, and is a slant toward a cover 3 side (positive Z-axis side) as it goes toward an edge.

The holder 2 has a through hole 25s having a circular shape in plan view in the vicinity of the center of the third slanted portion 25e of the blade-body holding recessed portion 25. In the connected state, the blade body 4 is visually recognized through the through hole 25s. On the blade body 4, letters, a symbol, or the like representing a type of the blade body 4 are shown at a position visible from the through hole 25s.

As shown in Figs. 8 and 9, the holder 2 has two finger rest portions 27a and 27b extending in the longitudinal direction on a surface (surface positioned on the negative Z-axis side) opposite to a side facing the cover 3. The finger rest portions 27a and 27b have a protruding shape extending in the longitudinal direction, and are provided side by side at intervals in the width direction. The holder 2 has, on a surface positioned on a positive Y-axis side, a finger rest portion 27c having a protruding shape and extending in the longitudinal direction (see Fig. 6). The finger rest portions 27a, 27b, and 27c have wavy irregularities in the longitudinal direction. These irregularities make it difficult for fingers placed on the finger rest portions 27a, 27b, and 27c to slide during use.

As shown in Figs. 12 and 13, as viewed from a side, the finger rest portion 27a has 27d and 27e that are formed in a notched shape recessed in the negative Z-axis side.

### [Cover 3]

The cover 3 is a member that is connected to the holder 2 by fitting, and sandwiches the blade body 4 together with the holder 2 in the connected state in which the cover 3 is connected to the holder 2 and the blade body 4. The cover tip-end portion 3a, which is a tip-end side of the cover 3, has the same shape in plan view as the holder tip-end portion 2a positioned on the tip-end side (positive X-axis side) of the blade-body holding recessed portion 25 of the holder 2 (see Fig. 11, Fig. 5, Fig. 3, and the like).

As shown in Figs. 10 and 11, the cover 3 has the through hole 31 through which the blade-body insertion portion 22 of the holder 2 is inserted in the connected state. The through hole 31 has an elongated hole shape similar to that of the through hole 46 of the blade body 4, and has a width (length in the Y-axis direction) changing in the longitudinal direction. Specifically, the through hole 31 has a first width portion 31a, a second width portion 31b, and a third width portion 31c corresponding to the first width portion 46a, second width portion 46b, and third width portion 46c of the through hole 46 of the blade body 4. For lengths of the widths, the first width portion 31a < the second width portion 31b < the third width portion 31c holds. The protruding portions 31d, 31e, and 31f protruding in the width direction are formed at a bottom of the through hole 31, on a side facing the holder 2 (see Fig. 11). These protruding portions 31d, 31e, and 31f are provided at positions corresponding to the recessed portions 22d, 22e, and 22f (see Figs. 5 and 6) of the holder 2, respectively.

As shown in Fig. 11, the cover 3 has a curved blade-body holding recessed portion 34 formed at a position facing the first slanted portion 25a and third slanted portion 25e of the holder 2. The curved blade-body holding recessed portion 34 is slanted along the first slanted portion 25a and third slanted portion 25e of the opposing holder 2, and has a shape partially recessed with respect to a holder facing surface 35 having a planar shape along the X-Y plane. The curved blade-body holding recessed portion 34 has edges 34a, 34b, and 34c along the second edge portion 44, first edge portion 45, and cutout portion 42c of the blade body 4.

As shown in Figs. 10 and 11, the cover 3 has the connecting protruding portions 32a, 32b, 32c, and 32d, and a hole portion 33. The hole portion 33 is a rectangular through hole extending in the longitudinal direction (X-axis direction). The connecting protruding portion 32d is a rectangular protruding portion provided adjacent to the hole portion 33 and extending in the longitudinal direction (X-axis direction), similarly to the hole portion 33. The connecting protruding portions 32a, 32b, and 32c are provided at positions corresponding to the connecting hole portions 23a, 23b, and 23c (see Fig. 4) of the holder 2. The connecting protruding portion 32d is provided at a position corresponding to the connecting hole portion 23d of the holder 2. In the connected state, the connecting protruding portions 32a, 32b, 32c, and 32d are inserted into the connecting hole portions 23a, 23b, 23c, and 23d of the holder 2, respectively, by which the holder 2 and the cover 3 are fitted to each other. A tip-end portion of the connecting protruding portion 32b has a hook shape, and the hook-shaped portion is engaged with the connecting hole portion 23b of the holder 2, by which the connecting protruding portion 32b does not come out of the connecting hole portion 23b.

A periphery of a position where the connecting protruding portion 32b is formed is the slanted edge portion 35a facing the cover-facing slanted surface 26a of the holder 2. The slanted edge portion 35a is slanted along the cover-facing slanted surface 26a of the holder 2. Specifically, the slanted edge portion 35a is a slanted surface directed in a direction away from the holder facing surface 35 (positive Z-axis side) toward an edge.

As shown in Figs. 1 to 3, the cover 3 has two finger rest portions 37a and 37b extending in the longitudinal direction on a surface (a surface positioned on the positive Z-axis side) opposite to a side facing the holder 2. Similarly to the finger rest portions 27a and 27b of the holder 2, the finger rest portions 37a and 37b have a protruding shape extending in the longitudinal direction, and are provided side by side at intervals in the width direction. The finger rest portions 37a and 37b have wavy irregularities in the longitudinal direction.

As shown in Figs. 12 and 13, as viewed from a side, the finger rest portion 37a has 37d and 37e that are formed in a notched shape recessed in the positive Z-axis side.

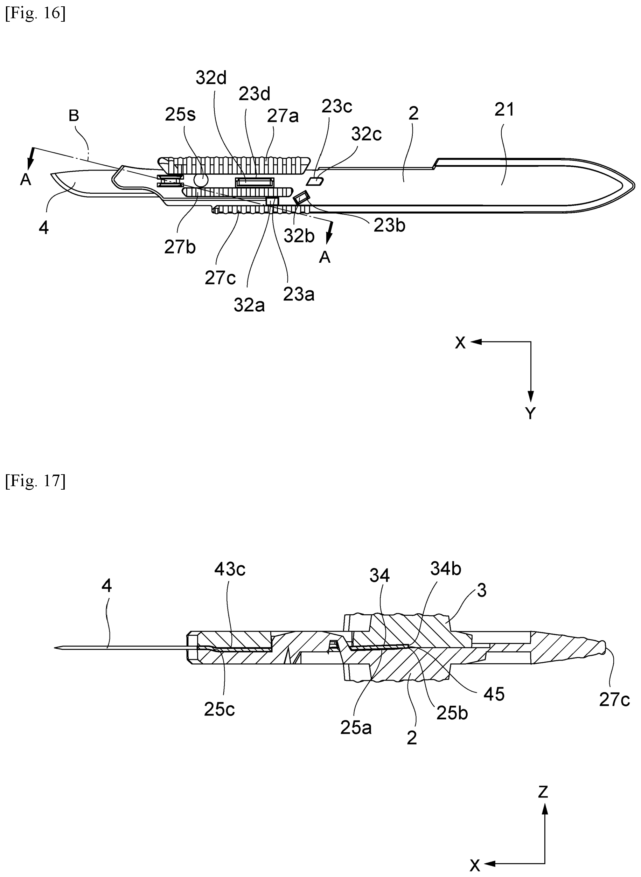

Fig. 16 is a plan view of the medical knife 1 from which the slider 5 is removed. Fig. 17 is a cross-sectional view taken along line A-A in Fig. 16. In Fig. 15, A-A is displayed at the same position as A-A in Fig. 16. The blade body 4 is sandwiched between the holder 2 and the cover 3 in a state in which the blade body 4 is curved in the thickness direction with a straight line B forming A-A as a center line of the curvature.

As shown in Fig. 17 which is a cross-sectional view, and Fig. 15 which is a plan view, the first edge portion 45 of the blade body 4 is sandwiched between the first slanted portion 25a of the holder 2 and the curved blade-body holding recessed portion 34 of the cover 3. More specifically, the first edge portion 45 of the blade body 4 abuts the first slanted portion 25a of the holder 2, and is curved toward the cover 3 side (negative Z-axis side) by stress.

As shown in Fig. 17 which is a cross-sectional view, and Fig. 15 which is a plan view, the slanted abutting portion 43c positioned in the vicinity of the center of the spine 43 of the blade body 4 is sandwiched between the second slanted portion 25c of the holder 2 and the cover tip-end portion 3a of the cover 3. More specifically, the slanted abutting portion 43c of the blade body 4 abuts the second slanted portion 25c of the holder 2, and is curved toward the cover 3 side (negative Z-axis side) by stress.

As shown in Fig. 17 which is a cross-sectional view, and Fig. 14 which is a side view, in the connected state, the blade body 4 is sandwiched by the holder 2 and the cover 3 in a state of being curved in the thickness direction so as to protrude toward the holder 2 side (so as to protrude toward the negative Z-axis side) along the straight line B connecting a central portion 25t of the holder 2 in a direction in which the first slanted portion 25a extends and a central portion 25u of the holder 2 in a direction in which the second slanted portion 25c extends. In other words, the straight line B representing the direction (second direction) in which blade body 4 is curved in the thickness direction is a straight line connecting a central portion of first edge portion 45 of blade body 4 and a central portion in a direction in which the slanted abutting portion 43c extends. From a positional relationship in which the first slanted portion 25a and the second slanted portion 25c are disposed, the straight line B is a straight line having a predetermined angle (30°) with respect to the X axis which is the longitudinal direction of the medical knife 1. This straight line B intersects with the through hole 46 of the blade body 4.

### [Slider 5]

As shown in Figs. 1, 2, and the like, the slider 5 is attached to the holder 2, the cover 3, and the blade body 4 connected to each other, and is slidable in the longitudinal direction (X-axis direction). As shown in Figs. 1 and 2, the slider 5 has two elongated holes 51a and 51b extending in the longitudinal direction on the surface positioned on the positive Z-axis side, and as shown in Fig. 8, the slider 5 has two elongated holes 52a and 52b extending in the longitudinal direction on the surface positioned on the negative Z-axis side. The finger rest portions 37a and 37b of the cover 3 are inserted into the elongated holes 51a and 51b, and the finger rest portions 27a and 27b of the holder 2 are inserted into the elongated holes 52a and 52b. As shown in Figs. 1, 2, 8, 12, and the like, the slider 5 has a side frame 53 formed sandwiched between the elongated holes 51a and 52a on a surface positioned on a negative Y-axis side. A knob 53a is formed in the vicinity of the center of the side frame 53 in the longitudinal direction. As shown in Fig. 7, the slider 5 has an elongated hole 54 in a surface positioned on the positive Y-axis side. The finger rest portion 27c of the holder 2 is inserted into the elongated hole 54.

At a position close to the knob 53a on the positive X-axis side, the side frame 53 of the slider 5 has protruding portions 53b and 53c protruding outward, that is, protruding toward the positive Z-axis side and the negative Z-axis side, respectively, in both the width directions. The protruding portions 53b and 53c are formed at positions facing each other. In the housed state in which the slider 5 covers the blade body 4 (see Figs. 12 and 1 and the like), the protruding portions 53b and 53c are engaged with recessed portions 37d and 27d, respectively. In the use state in which the slider 5 has moved from the housed state to expose the blade body 4 (see Figs. 13 and 2 and the like), the protruding portions 53b and 53c are engaged with recessed portions 37e and 27e, respectively.

The slider 5 has a through hole 5a through which the blade body 4 can be inserted during sliding (see Fig. 7).

### [Use of medical knife 1]

When changing the medical knife 1 from the housed state to the use state, the user slides the slider 5 toward the negative X-axis side while pinching and pulling up the knob 53a of the slider 5 with the fingers. At this time, engagement between the protruding portions 53b and 53c of the slider 5 and the recessed portions 37d and 27d is released, and the slider 5 becomes slidable. When the slider 5 is moved to the negative X-axis side, the protruding portions 53b and 53c of the slider 5 are engaged with the recessed portions 37e and 27e, and further movement of the slider 5 to the negative X-axis side is restricted. Accordingly, the medical knife 1 is in the use state in which the blade body 4 is exposed (see Fig. 2). Note that the movement of the slider 5 is restricted by the engagement between the protruding portions 53b and 53c and the recessed portions 37e and 27e, and by longitudinal edges of the finger rest portions 27a, 27b, 37a, and 37b abutting, edge portions of the elongated holes 52a, 52b, 51a, and 51b, respectively.

When the medical knife 1 is used in the use state, for example, the user grips around the finger rest portions 27a, 27b, 27c, 37a, and 37b with a thumb and an index finger. At this time, the grip part 21 is gripped by a middle finger, a ring finger, and a little finger to stabilize the medical knife 1 so that the medical knife 1 does not wobble. Note that how the medical knife 1 is held may change depending on the user and a use situation.

### [Modification]

The medical knife 1 of the present invention is not limited to the shape described in the above embodiment, and may be partially modified from the shape of the embodiment without departing from the gist of the invention.

The angles θ1 and θ2 of the blade body 4 may be arbitrarily changed. That is, the angle θ1 is not limited to 120°, and the angle θ2 is not limited to 90° (right angle). In this case, the angle formed by the extended line of the spine 43 and the extended line of the first edge portion 45 is also not limited to 30°. However, in order to dispose the positions of the first slanted portion 25a and the second slanted portion 25c so as to stably sandwich the blade body 4, the angle formed by the extended line of the spine 43 and the extended line of the first edge portion 45 is preferably 10° or greater and 80° or less, and the angles θ1 and θ2 are preferably determined such that the angle formed by the extended line of the spine 43 and the extended line of the first edge portion 45 is an angle described above. Note that it is preferable that the angles θ1 and θ2 be set to the angles shown in the embodiment, because the second direction, which is a direction in which the blade body 4 is curved, can be set to an appropriate direction, while a shape of an end portion of the blade body 4, which is on a side opposite to the tip 41, is relatively compact. In addition, even in a configuration in which the angle formed by the extended line of the spine 43 and the extended line of the first edge portion 45 is 10° or greater and 80° or less as described above, the first slanted portion 25a extends in a direction different from a direction in which the second slanted surface 25c extends, by which the blade body 4 can be pressed to be curved in two different directions. Accordingly, wobble of the blade body 4 can be effectively prevented.

In the blade body 4, each of the blade edge 42 and spine 43 has a partially curved shape, but at least either the blade edge 42 or the spine 43 may have a linear shape.

The blade body 4 may not necessarily have the second edge portion 44, and the first edge portion 45 may connect the spine 43 and blade edge 42. In addition to the first edge portion 45 and the second edge portion 44, the blade body 4 may have another edge portion. Even in such a case, if a positional relationship between the blade body 4, and the first slanted portion 25a and the second slanted portion 25c is appropriate, the blade body 4 can be stably sandwiched.

### [Features of medical knife 1]

The medical knife 1 holds the blade body 4 with the holder 2 and the cover 3. The holder 2 and the cover 3 sandwich the blade body 4 while curving the blade body 4 in the thickness direction (Z-axis direction) along a direction of a curvature intersecting the X-axis direction, which is a direction in which the spine 43 of the blade body 4 extends in plan view, and having a predetermined angle with respect to the X-axis direction.

According to such a medical knife 1, the holder 2 and the cover 3 sandwich the blade body 4 while curving the blade body 4 in the thickness direction (Z-axis direction) along a direction of a curvature not parallel to the spine 43 of the blade body 4 and having a predetermined angle. Therefore, it is possible to prevent wobble of the blade body 4 not only in the longitudinal direction of the blade body 4 but also in the width direction of blade body 4 at the same time. Accordingly, it is possible to provide the medical knife 1 capable of accurately incising a target site or the like.

In addition, in the medical knife 1, the blade body 4 has the through hole 46 having an elongated hole shape extending parallel with the X-axis direction in which the spine 43 extends, and formed in the thickness direction (Z-axis direction). The holder 2 has the blade-body insertion portion 22 having a protruding shape and being inserted into the through hole 46. The through hole 46 of the blade body 4 is formed so as to intersect the second direction in which the blade body 4 is curved. Accordingly, the direction in which the blade body 4 is curved intersects the through hole 46 through which the blade-body insertion portion 22 is inserted, and thus the blade body 4 can be held more stably with respect to the holder 2 and the cover 3. Accordingly, it is possible to provide the medical knife 1 that more effectively prevents wobble of the blade body 4.

In the medical knife 1, the blade body 4 has, as the outer edges thereof, the blade edge 42, the spine 43, and the first edge portion 45 in plan view. The blade edge 42 is a portion extending from the tip 41 and having a cutting edge at least partially. The spine 43 is a portion extending from the tip 41. The first edge portion 45 extends in a direction having the first angle (for example, 30°) with respect to the spine 43. The holder 2 has a blade-body holding recessed portion 25 having a recessed shape and formed along a shape of the blade body 4. The blade-body holding recessed portion 25 has the first slanted portion 25a and the second slanted portion 25c. The first slanted portion 25a is slanted in a direction perpendicular to the first edge portion 45 in plan view, at the position where the first edge portion 45 abuts. The second slanted portion 25c is slanted in a direction perpendicular to the spine 43 in plan view, at the position where the spine 43 abuts. Thus, because the medical knife 1 has a configuration in which the blade body 4 is curved in the thickness direction by the first slanted portion 25a and the second slanted portion 25c of the blade-body holding recessed portion 25, it is possible to prevent wobble in the longitudinal direction, width direction, and rotation direction of the blade body with a relatively simple configuration. Therefore, the medical knife 1 is a medical knife capable of accurately incising a target site or the like.

In addition, in the medical knife 1, the blade body 4 has, in plan view, the second edge portion 44 extending in a direction having a second angle (for example, 120°) with respect to the spine 43 from an end portion 43d of the spine 43, the end portion 43d being on a side opposite to the tip 41. The first edge portion 45 extends, in plan view, from the second edge portion 44 toward the blade edge 42, in a direction having a third angle (for example, 90°) with respect to the second edge portion 45. With such a configuration, the second slanted portion 25c and the first slanted portion 25a are provided on the spine 43 and the first edge portion 45 connected to the second edge portion 44 continuing from the spine 43, respectively, and thus the blade body 4 can be sandwiched more stably by the holder 2 and the cover 3.

In addition, in the medical knife 1, in plan view, the through hole 46 of the blade body 4 is formed so as to intersect the straight line B connecting the central portion in the direction in which the first slanted portion 25a extends and the central portion in the direction in which the second slanted portion 25c extends. According to such a medical knife 1, the blade body 4 can be more stably held with respect to the holder 2 and the cover 3, and wobble of the blade body 4 can be more effectively prevented.

### Reference Signs List

1 Medical knife
2 Holder
2a Holder tip-end portion
21 Grip part
22 Blade-body insertion portion
22d, 22e Recessed portion
23a, 23b, 23c, 23d Connecting hole portion
25 Blade-body holding recessed portion
25a First slanted portion
25c Second slanted portion
25e Third slanted portion
25s Through hole
26 Cover facing surface
26a Cover-facing slanted surface
27a, 27b, 27cFinger rest portion
3 Cover
3a Cover tip-end portion
31 Through hole
31d, 31e Protruding portion
32a, 32b, 32c, 32d Connecting protruding portion
33 Hole portion
34 Curved blade-body holding recessed portion
34a, 34b Edge portion
35 Holder facing surface
35a Slanted edge portion
37a Finger rest portion
37d Recessed portion
37e Recessed portion
4 Blade body
41 Tip
42 Blade edge
42a Curved blade edge
42b Straight blade edge
42c Cutout portion
43 Spine
43a Curved spine
43b Straight spine
43c Slanted abutting portion
44 Second edge portion
45 First edge portion
46 Through hole
5 Slider
5a Through hole
51a, 51b, 52a, 52b, 54 Elongated hole
53 Side frame
53a Knob
53b, 53c Protruding portion

## Claims

1. A medical knife that sandwiches a blade body with a holder and a cover, wherein
the blade body has, in plan view,
a blade edge extending from a tip, and having a cutting edge at least partially,
a spine extending from the tip, and
a first edge portion extending in a direction having a first angle with respect to the spine,
the holder has a blade-body holding recessed portion having a recessed shape and formed along a shape of the blade body, and
the blade-body holding recessed portion has
a first slanted portion slanted in a direction perpendicular to the first edge portion in plan view at a position where the first edge portion abuts, and
a second slanted portion slanted in a direction perpendicular to the spine in plan view at a position where the spine abuts.

2. The medical knife according to claim 1, wherein
the blade body further has, in plan view, a second edge portion extending in a direction having a second angle with respect to the spine, from an end portion of the spine, the end portion being on a side opposite to the tip, and
the first edge portion extends, in plan view, from the second edge portion toward the blade edge, in a direction having a third angle with respect to the second edge portion.

3. The medical knife according to claim 2, wherein
the third angle is 90°.

4. The medical knife according to claim 1, wherein
the blade body has a through hole having an elongated hole shape extending parallel with a direction in which the spine extends, and formed in a thickness direction,
the holder has a blade-body insertion portion having a protruding shape and being inserted into the through hole, and
in plan view, the through hole is formed so as to intersect a straight line connecting a central portion in a direction in which the first slanted portion extends and a central portion in a direction in which the second slanted portion extends.
